# FASCICULE DE BREVET EUROPEEN

(11) **EP 1 830 893 B1**
(45) Date de publication et mention de la délivrance du brevet: **27.03.2013**
(21) Numéro de dépôt: 06709036.5
(22) Date de dépôt: 05.01.2006
(51) Int. Cl.: A61L 9/12

(54) **DISPOSITIF DE DIFFUSION MULTI-ODEURS**
VORRICHTUNG ZUR ABGABE VON MEHREREN DÜFTEN
MULTI-FRAGRANCE DIFFUSION DEVICE

(30) Priorité: 05.01.2005 FR 0500079
(43) Date de publication de la demande: 12.09.2007
(73) Titulaire: Conception et Valorisation de Brevets (CVB), 75012 Paris (FR)
(72) Inventeur: SUISSA, David, F-75012 Paris (FR); JEANJEAN, Clément, F-75007 Paris (FR)
(74) Mandataire: Novagraaf Technologies
(86) Numéro de dépôt international: PCT/FR2006/000019
(87) Numéro de publication internationale: WO 2006/072744

(56) Documents cités:
- DE-A1- 10 044 894
- FR-A- 2 815 294
- US-A- 5 178 327
- US-A- 5 565 148
- US-A1- 2002 158 351

## Description

La présente invention se rapporte au domaine de la diffusion d'odeurs.

L'invention concerne plus particulièrement un procédé et un dispositif de diffusion d'une ou plusieurs odeurs piloté électroniquement. Elle est particulièrement adaptée pour odoriser des volumes de la dimension d'une salle de taille moyenne, c'est-à-dire dont la surface est comprise entre 20 et 100 mètres carrés.

Différentes techniques de diffusion d'odeurs sont connues de l'état de l'art. La plus courante consiste en l'utilisation de matériaux imbibés de molécules odorantes qui se libèrent dans l'atmosphère par convection naturelle. Cette technique est largement mise en oeuvre pour odoriser les habitacles d'automobiles par exemple. Une meilleure efficacité de la convection peut être obtenue en forçant la circulation d'un flux d'air passant au contact du matériau imbibé. Dans ce cas, le flux d'air est créé généralement au moyen d'une turbine électrique. Une autre technique courante consiste à faire brûler une bougie parfumée ou un matériau tel que l'encens. La forte chaleur provoquée par la combustion accélère la libération des molécules odorantes. Enfin des dispositifs d'odeurs utilisent la technique de micronisation d'un liquide olfactif qui consiste à diffuser celui-ci en fines gouttelettes qui se vaporisent rapidement en libérant les molécules odorantes. Cette technique est celle généralement employée pour odoriser de grands espaces, comme des halls de gare par exemple. Quelle que soit la technique mise en oeuvre par le dispositif de diffusion d'odeur, il convient toujours de ré-imbiber ou remplacer un matériau imbibé de molécules odorantes lorsque celui-ci s'est desséché, ou de remplir ou remplacer un flacon contenant un liquide odorant lorsque celui-ci s'est vidé.

Certains diffuseurs d'odeurs sont pilotés par un dispositif électronique. Le brevet EP01212104B1 décrit un dispositif de diffusion de parfum basé sur un flux d'air passant au contact d'un matériau imbibé, dispositif piloté par un poste de télévision numérique ou par un micro-ordinateur. Le brevet EP01054697B1 présente un dispositif permettant de diffuser plusieurs parfums avec la particularité d'éviter la rémanence lors d'un changement d'odeur grâce à une capacité d'obturation hermétique des réservoirs de parfums que l'on ne souhaite pas diffuser. Ce document décrit un réservoir de forme globalement sphérique qu'il convient de faire tourner d'un quart de tour pour placer ou non le matériau odorant dans un flux d'air.

On connaît également, par le brevet US 5 565 148, un dispositif de diffusion d'odeurs présentant un couloir de circulation d'air au milieu duquel un barillet vient présenter un support d'odeurs. L'air circulant dans le couloir traverse le support emmenant avec lui des molécules odorantes.

L'inconvénient des solutions où les supports d'odeurs sont disposés sur des supports cylindriques du type barillet réside dans le fait que le passage d'une odeur à une autre est laborieux et coûteux en temps, notamment si les deux supports sont opposés dans le barillet. Cela nécessite parfois plusieurs secondes.

On connaît également, par la demande de brevet FR 2 815 294, un dispositif de chauffage/climatisation automobile comprenant des éléments de diffusion olfactive. Une enveloppe comprenant des passages d'air est disposée sur le trajet de l'air, à l'intérieur de laquelle un support coulisse pour positionner un agent aromatique en regard des passages. Cette solution reste limitée à un nombre d'odeurs limité pour être opérationnelle. De plus, la solution présente des pertes de charge importantes en raison de la position excentrée de l'enveloppe par rapport au chemin du flux d'air.

On connaît également des cartouches de parfum liquide activées par un dispositif à bras pour permettre l'ouverture de celle-ci et l'évaporation des molécules (US 2002 / 0 158 351), des cartouches d'odeurs constituées d'un matériau poreux ou similaire à une éponge pour emprisonner les molécules odorantes (US 5 565 148), des cartouches insérées dans une ouverture réalisée sur un dispositif de diffusion d'odeurs, ces cartouches étant constituées de gel ou imbibées de substances odoriférantes (DE 100 44 894) et des cartouches cylindriques constituées d'une structure rigide présentant des quartiers de cylindre aptes à recevoir des substances olfactives (US 5 178 327).

L'art antérieur ne permet pas de concevoir des dispositifs multi odeurs pilotés électriquement qui soient à la fois de conception simple, modulaires et aisément rechargeables. L'invention vise notamment à concevoir des dispositifs multi odeurs ne nécessitant qu'une seule turbine électrique quel que soit le nombre d'odeurs que l'on souhaite diffuser, et présentant un unique orifice diffuseur. Elle autorise des conceptions modulaires dans lesquelles des modules identiques sont juxtaposés simplement les uns contre les autres, chaque module pouvant diffuser une odeur. Ainsi concevoir un dispositif devant diffuser une odeur supplémentaire s'obtient simplement par l'ajout d'un module à une conception déjà réalisée. L'invention vise de surcroît à faciliter la recharge ou le remplacement des matériaux odorants une fois ceux-ci asséchés grâce à la mise en oeuvre de cartouches aisément remplaçables. Ces avantages, ainsi que d'autres qui apparaîtront à la lecture de la description ci-après, font que l'invention est particulièrement adaptée pour diffuser plusieurs odeurs dans des salles de dimension moyenne, entre 20 et 100 m2, tandis que l'état de l'art s'adresse essentiellement à odoriser soit de plus petits volumes tels que l'habitable d'un véhicule, soit de plus grands volumes tels que des halls de gare au moyen le plus souvent d'une seule odeur.

L'invention porte sur un dispositif de diffusion d'une ou plusieurs odeurs à partir d'un ou plusieurs matériaux imbibés de molécules odorantes volatiles au contact desquels circule un flux d'air. Dans le cas d'une diffusion multi odeurs, le dispositif est composé d'une pluralité de modules identiques, un module par odeur que l'on souhaite diffuser, et d'une turbine électrique générant un flux d'air. La diffusion ou l'arrêt de diffusion d'une odeur, ainsi que le choix de l'odeur diffusée, sont commandés électroniquement. L'invention permet de ré imbiber ou remplacer facilement les matériaux odorants une fois ceux-ci asséchés, grâce à la mise en oeuvre de cartouches amovibles aisément remplaçables ou rechargeables. Elle évite la rémanence d'une odeur à la fin de sa diffusion, ainsi que le dessèchement spontané des cartouches odorantes, grâce à un positionnement en enceinte hermétique des cartouches dont l'utilisateur ne souhaite pas diffuser l'odeur.

À cet effet, l'invention concerne, dans son acception la plus générale, un dispositif de diffusion d'odeurs comprenant des moyens de génération d'un flux d'air et une sortie, lesdits moyens de génération et la sortie définissant entre eux un chemin pour le flux d'air, le dispositif étant tel qu'il comprend ;
- une pluralité de modules de diffusion d'odeurs, chaque module comprenant :
   o un carter dans lequel est pratiquée une fenêtre,
   o un coulisseau, dans lequel sont pratiquées ;
      ■ au moins une première fenêtre destinée à accueillir une source de molécules odorantes ; et
      ■ une deuxième fenêtre ;
      la coulisseau coulissant à l'intérieur du carter entre :
- une position de diffusion dans laquelle ladite première fenêtre du coulisseau est en regard de la fenêtre du carter ; et
- une position non odorante dans laquelle ladite deuxième fenêtre du coulisseau est en regard de la fenêtre du carter ;
les modules étant positionnés de manière à ce que les axes des fenêtres des carters des différents modules soient alignés sur ledit chemin pour le flux d'air ;
- des moyens permettant d'amener individuellement les coulisseaux sur l'une desdites positions ;
de sorte que le flux d'air traverse successivement chaque module par la fenêtre du carter ainsi que par l'une des première et deuxième fenêtres du coulisseau selon la position de celui-ci.

Le chemin du flux d'air entre les moyens de génération du flux d'air et la sortie peut être matérialisé par l'utilisation de parois de délimitation, par exemple un tuyau. On entend par " alignés " le fait que les axes des fenêtres des carters (c'est-à-dire l'axe de traversée du flux d'air) sont sensiblement parallèles à la tangente du chemin du flux d'air à l'emplacement où le module est implanté. Ce parallélisme implique favorablement un angle de moins de 30° entre l'axe de la fenêtre et la tangente. Particulièrement, il sera choisi un angle de moins de 10°. En outre, les modules sont disposés afin de centrer les fenêtres des carters sur l'axe central du chemin, ainsi ces fenêtres des carters sont pleinement traversées par le flux d'air.

L'utilisation de modules d'odeurs est avantageuse en ce qu'elle permet de chaîner aisément un grand nombre de modules en vue de diffuser de nombreuses odeurs. L'invention permet à ce titre de proposer une solution modulaire efficace là où les solutions de l'art antérieur restent limitées à un nombre restreint de compartiments (nombre d'éléments d'un barillet par exemple). Le chaînage linéaire type " lego " permet de n'augmenter qu'une seule dimension (la longueur) pour augmenter le nombre d'odeurs, en conservant constantes la hauteur et la largeur du dispositif de diffusion multi-odeurs contrairement à un barillet cylindrique par exemple. La modularité permet également des substitutions de modules aisées et/ou des changements fréquents (forte consommation ou besoin de nouvelles senteurs).

La présence de moyens de soufflerie, type ventilateur ou turbine électrique, présente l'avantage de diffuser rapidement et précisément les odeurs à l'opposé des solutions basées sur la convection par chauffage qui sont soumises à un processus de diffusion non guidée dans l'atmosphère. Ainsi l'utilisateur sent rapidement les odeurs.

Une telle solution est également très économique au vu du nombre d'odeurs diffusables grâce à l'utilisation de modules similaires (d'où des économies d'échelle) et d'une seule source de flux d'air.

Dans un mode de réalisation, ledit chemin présente des parois formant un couloir d'air et lesdites fenêtres desdits carters sont alignées dans le couloir d'air. Ainsi la solution apparaît compacte : un seul point d'entrée en air et un seul point de sortie sont nécessaires pour toutes les odeurs. Cela permet notamment d'être très directif sur les odeurs et de ne pas obliger l'utilisateur à se déplacer pour sentir une autre odeur. Cela est notamment pratique pour la diffusion d'odeurs pendant des jeux vidéo ou des vidéos alors que l'utilisateur est assis dans son canapé.

Le couloir d'air est entendu comme un ensemble de parois qui délimitent efficacement le chemin parcouru par le flux d'air. Un tel couloir peut être mis en oeuvre par l'intermédiaire d'un tuyau ou de plusieurs sections de tuyaux. Par exemple, une première section de tuyau guide le flux d'air entre les moyens de génération d'air et un premier module, une deuxième section guide le flux d'air ayant traversé le module vers un deuxième module, etc. pour l'ensemble des modules disposés sur le chemin d'air, et enfin une dernière section de tuyau guidant le flux d'air sortant du dernier module (après avoir traversé l'ensemble des modules du dispositif de diffusion) vers la sortie du dispositif.

La présence du couloir d'air aux fins de confinement et de guidage du flux d'air permet de diminuer considérablement les pertes de charge du flux d'air.

Particulièrement, lesdites fenêtres sont sensiblement de la dimension de la section dudit couloir d'air (au moins 50%, de préférence entre 70 et 100% de la section du couloir) et sont orientées sensiblement perpendiculairement à l'axe de ce couloir. Dans cette configuration, l'utilisation du flux d'air est optimisée afin de capter le maximum de molécules odorantes.

Afin de fournir une grande compacité et une étanchéité des modules entre eux, ceux-ci sont juxtaposés carter contre carter.

Selon différents modes de réalisation :
- les moyens permettant de déplacer les coulisseaux sont actionnés par un organe de commande électrique,
- le dispositif comprend une pluralité de moyens permettant de déplacer les coulisseaux, chacun de ladite pluralité de moyens étant associé individuellement à l'un de ladite pluralité de modules de diffusion d'odeurs.

Particulièrement, les moyens permettant de déplacer le coulisseau sont un servomoteur dont le stator est solidaire du carter et dont le rotor est solidaire d'un doigt excentré qui coopère avec une rainure pratiquée dans le coulisseau.

L'utilisation de commandes électriques (via une carte et un pilotage électroniques) confère au dispositif une grande aptitude à changer rapidement d'odeurs et à conserver une vitesse constante pour passer d'une odeur à une autre à l'opposé des solutions antérieures, par exemple pour des odeurs disposées sur un cylindre rotatif. Avec par exemple 8 odeurs, il faut faire 4 pas de rotations d'une odeur à l'odeur située physiquement à l'opposé sur le cylindre, ce qui nécessite approximativement quatre fois le temps nécessaire pour passer d'une odeur à une odeur adjacente.

La carte électronique de pilotage ou le programme pilotant ladite carte peut recevoir des paramètres de l'utilisateur déterminants dans la stratégie de pilotage, par exemple la dimension de la pièce à odoriser. À cet effet, une liaison parallèle ou série (USB - Universal Sériai Bus, RS232 ou autre) peut équiper la carte électronique pour paramétrer son pilotage à partir d'une source de contrôle extérieure.

Selon un mode de réalisation, chacun des carters présente globalement une forme en U à l'intérieur de laquelle s'insère et coulisse le coulisseau associé au carter.

Afin de garantir l'étanchéité des modules et diminuer ainsi l'assèchement inutile des cartouches, chaque module n'ayant pas de voisin sur la face où se trouve visible la cartouche comprend une plaque apte à enfermer de manière inaccessible à l'air la source lorsque le coulisseau se trouve en position telle que la deuxième fenêtre se trouve en regard de la fenêtre du carter.

Lors des opérations de maintenance (changement de module ou de cartouche par exemple), les modules peuvent être manipulés. Afin d'éviter que les coulisseaux ne soient déplacés malencontreusement, les modules qui sont amovibles comprennent, en outre, des moyens de blocage des coulisseaux dans la position non odorante lorsqu'ils ne sont pas insérés dans le dispositif. Ce dernier comprend alors des moyens de déblocage desdits moyens de blocage lorsque le module amovible est inséré dans le dispositif.

Selon un mode de réalisation, lesdits modules comportent des moyens de stockage de données (par exemple une puce électronique du type carte SIM ou RFID ou un code barre) comprenant au moins des données numériques représentatives d'informations concernant les sources de molécules odorantes qu'ils renferment, et le dispositif comprend, en outre, des moyens de lecture desdits moyens de stockage de données, ces moyens de lecture étant reliés à une carte électronique de pilotage du dispositif.

Selon une variante à ce mode de réalisation dans laquelle ladite source de molécules odorantes est une cartouche amovible imbibée de molécules odorantes, ladite cartouche comprend des moyens de stockage de données comprenant au moins des données numériques représentatives de l'odeur contenue dans la cartouche et le dispositif comprend, en outre, des moyens de lecture desdits moyens de stockage de données, ces moyens de lecture étant reliés à une carte électronique de pilotage du dispositif.

La présence de telles puces électroniques est avantageuse en ce qu'elle permet à l'unité de traitement (carte électronique) de disposer d'informations essentielles au bon fonctionnement du dispositif. On note les informations relatives à la nature de l'odeur contenue dans chaque cartouche afin de commander efficacement des actionneurs (servomoteurs) appropriés et également les informations relatives à l'état d'assèchement des cartouches (ou leur utilisation) afin d'adapter le flux d'air requis pour assurer une odeur constante dans le temps et de signaler, si nécessaire, le changement d'une cartouche. Il est ainsi possible de contrôler finement la diffusion des odeurs en fonction des performances olfactives du nez, à l'image du contenu de la demande de brevet PCT/FR05/050968.

Selon le mode de réalisation choisi, lesdits moyens de génération d'un flux d'air sont choisis parmi un ventilateur et une turbine à air.

Afin de compenser un couloir d'air de grande dimension et les pertes de charge inévitables, il peut être prévu que le dispositif comprend, en outre, des moyens d'accélération du flux d'air disposés dans le couloir d'air et pilotés par une carte électronique de pilotage du dispositif.

Également, le dispositif peut comprendre, en outre, des moyens d'adaptation du débit du flux d'air en sortie dudit couloir d'air. Une turbine électrique à air peut être utilisée à cet effet et permet de contrôler les paramètres de sortie du flux d'air, notamment pour gérer la distance à laquelle on envoie les molécules parfumées. La turbine en entrée du couloir d'air permet, quant à elle, de gérer la quantité de molécules parfumées arrachées des cartouches. Ces turbines sont pilotées par la carte électronique selon les paramètres saisis par l'utilisateur (taille de la pièce par exemple).

Il est également prévu que le coulisseau d'au moins un module comprend, en outre, au moins une troisième fenêtre agencée pour accueillir une deuxième source de molécules odorantes, lesdits moyens permettant de déplacer le coulisseau étant alors aptes à amener le coulisseau sur l'une des au moins trois positions correspondant à l'alignement d'une fenêtre du coulisseau avec la fenêtre du carter. Le nombre d'odeurs diffusables pouvant être considérablement accru. De tels coulisseaux avec trois ou plus fenêtres sont utiles pour des odeurs très utilisées pour lesquelles il y aurait lieu de changer les cartouches fréquemment. À l'aide des puces électroniques, la carte électronique de pilotage est informée de la nécessiter de piloter les servomoteurs de façon adéquate pour positionner le coulisseau dans les trois positions possibles.

L'invention a également pour objet un dispositif de diffusion d'odeurs tel qu'il comprend en outre une structure rigide comprenant des moyens de réception agencés pour disposer 2, 4 ou 8 modules de diffusion d'odeurs dans la structure rigide, la structure rigide étant agencée pour coopérer avec les moyens de réception de telle sorte que les fenêtres des modules de diffusion d'odeurs soient alignées avec un conduit d'air pourvu dans le dispositif, pour former un ensemble modulaire.

Dans un mode de réalisation, l'ensemble modulaire comprend des moyens de blocage agencés pour bloquer les coulisseaux dans la position non odorante lorsque ledit ensemble n'est pas inséré dans le dispositif et pour débloquer lesdits coulisseaux lorsqu'ils coopèrent avec des moyens de déblocage pourvus sur ledit dispositif.

De tels ensembles modulaires simplifient fortement les procédures de maintenance des dispositifs de diffusion d'odeurs. En effet, l'utilisateur n'a pas besoin d'accéder jusqu'à la cartouche mais seulement substituer un ensemble modulaire par un autre. En outre, des ensembles " thématiques " peuvent être développés et associés à un film précis de telle sorte que l'utilisateur insère le module approprié dans le dispositif avant de visionner son film. Les opérations sont donc simplifiées.

L'invention a également pour objet un procédé de diffusion d'odeurs mettant en oeuvre le dispositif tel que précédemment évoqué et comprenant :
- une étape d'insertion de cartouches d'odeur à l'intérieur des premières fenêtres des modules;
- une étape de création d'un flux d'air au moyen desdits moyens de production d'un flux d'air, ledit flux d'air traversant l'ensemble des fenêtres des carters,
- une étape de présentation d'au moins une première fenêtre en vis-à-vis de la fenêtre d'au moins un carter de telle sorte que le flux d'air traversant les fenêtres des carters traverse également la cartouche d'odeur contenue dans ladite première fenêtre présentée ce qui diffuse l'odeur de celle-ci.

Dans un mode de réalisation, le procédé comprend, en outre et lors de l'utilisation d'une cartouche, une étape de mise à jour d'une puce électronique associée à ladite cartouche et comprenant au moins des données numériques représentatives de l'utilisation de la cartouche.

Il est également prévu de diffuser plusieurs odeurs en même temps, nécessitant :
- soit la mise en position odorante simultanée de plusieurs coulisseaux le long du couloir d'air,
- soit une commutation très rapide des coulisseaux des odeurs à diffuser par les moyens de pilotage. Cette rapidité est possible avec la technologie proposée à savoir le pilotage électronique des servomoteurs.

Il est très avantageux de constituer la cartouche comme épousant au maximum les parois de la fenêtre l'accueillant. En effet, cette caractéristique, additionnée à la présence du couloir d'air confinant ce dernier, assure que l'intégralité du flux d'air vient lécher les billes parfumées, offrant de la sorte une grande précision sur la quantité de parfums diffusés : les billes sont réparties sur 100% de la section du couloir d'air pour avoir un contrôle fin de la diffusion et de la perte de charge dans les tuyaux. On arrache ainsi les molécules de manière fine : cela relève notamment de la mécanique des fluides sur un conduit de section presque constante. Cette précision permet notamment de suivre efficacement l'état d'assèchement des cartouches imbibées pour adapter le flux d'air et signaler le besoin de changement de la cartouche.

L'invention va être expliquée en référence aux figures 1 à 10 annexées qui présentent une conception de dispositif mono-odeur et une conception de dispositif multi-odeurs selon l'invention, parmi lesquelles :
- la figure 1 représente un module diffuseur d'une odeur, dans une position ne diffusant pas d'odeur ;
- la figure 2 représente ce même module diffuseur dans une position diffusant une odeur ;
- la figure 3 représente une vue en coupe d'un module diffuseur dans une position ne diffusant pas d'odeur ;
- la figure 4 représente un dispositif de diffusion de quatre odeurs mettant en oeuvre l'invention ;
- la figure 5 représente un ensemble de modules amovible conforme à la présente invention ;
- les figures 6 à 8 illustrent les mécanismes de blocage des coulisseaux de la présente invention ;
- la figure 9 représente une vue de trois-quarts du dispositif de diffusion d'odeurs selon l'invention ; et
- la figure 10 représente le même dispositif capot enlevé montrant l'architecture interne du dispositif.

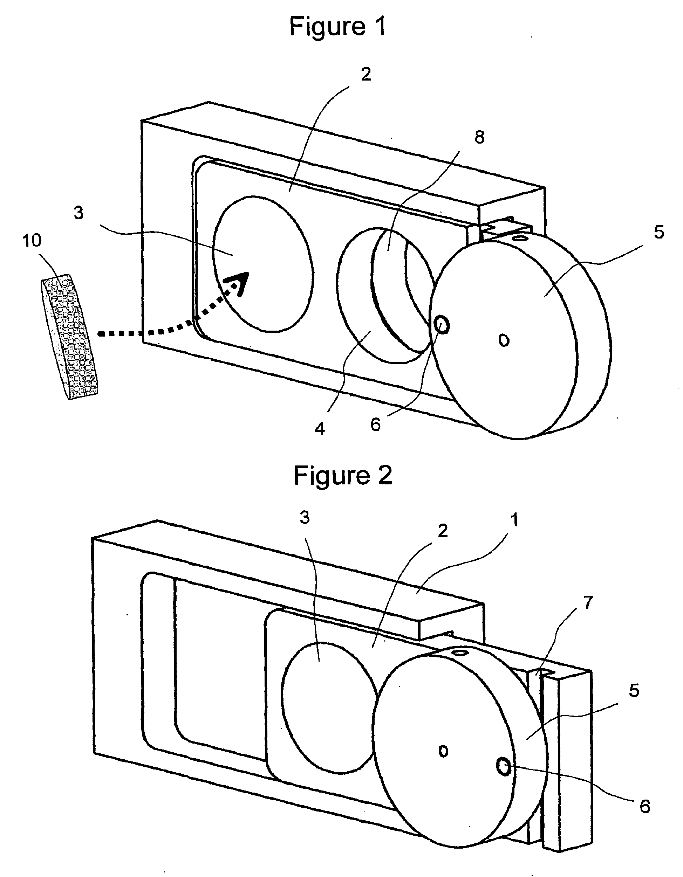

En référence aux figures 1 et 2, selon un mode de conception de l'invention, chaque module est composé d'un carter 1 à l'intérieur duquel coulisse un coulisseau 2. Le coulisseau 2 comporte deux fenêtres circulaires 3 et 4 de diamètres sensiblement égaux traversant le coulisseau de part en part. La fenêtre 3 est destinée à accueillir une cartouche amovible 10 contenant des matériaux imbibés de molécules odorantes volatiles. Un moteur électrique 5, par exemple un servomoteur, dont le stator est solidaire du carter 1, permet de déplacer le coulisseau entre deux positions extrêmes. Pour cela la partie tournante du moteur 5 comporte un doigt 6 qui coopère avec une rainure 7 pratiquée dans le coulisseau 2. Dans l'une des deux positions du coulisseau 2, qualifiée par la suite de position non odorante et illustrée par la figure 1, la fenêtre 4 du coulisseau 2 se trouve en regard d'une fenêtre 8 de diamètre sensiblement égal pratiquée dans le carter 1. Dans l'autre position du coulisseau 2, qualifiée de position odorante et illustré par la figure 2, c'est la fenêtre 3 accueillant la cartouche de matériaux odorants 10 qui se trouve en regard de la fenêtre 8 du carter 1.

Si de l'air sous pression est amené à l'intérieur de la fenêtre 8 du carter, l'air traversera celle-ci et le coulisseau 2 par l'une des fenêtres 3 ou 4 selon que le coulisseau 2 est en position odorante ou non odorante. C'est seulement si l'air traverse la fenêtre 3 dotée d'une cartouche d'odeurs qu'il se chargera de molécules odorantes.

Dans un mode de réalisation, le carter 1 présente une forme globalement en U ce qui facilite la réalisation et permet aisément d'y insérer le coulisseau 2.

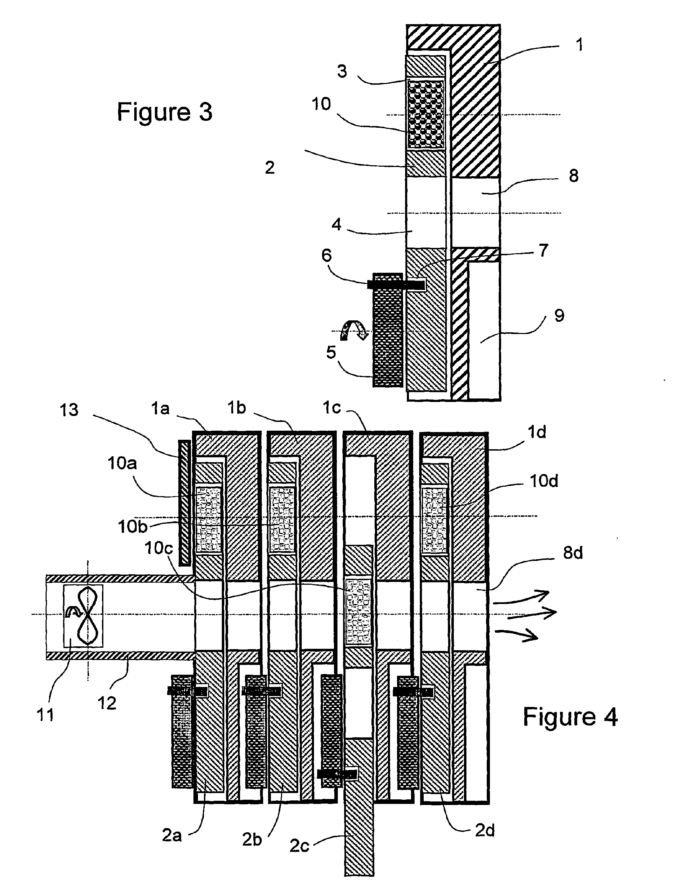

La figure 3 représente une vue en coupe d'un module dans une position non odorante. La cartouche 10 remplie de matériaux imbibés de molécules odorantes est en place dans la fenêtre 3 du coulisseau 2. Un évidement 9 est réalisé dans le carter 1 en vue de permettre la conception d'un dispositif diffusant plusieurs odeurs par la simple juxtaposition de plusieurs modules, le moteur 5 d'un module s'emboîtant dans l'évidement 9 du module voisin.

La figure 4 représente une conception de dispositif mettant en oeuvre l'invention composé de quatre modules désignés a, b, c et d. Ces modules comprennent respectivement des carters 1 a, 1 b, 1 c et 1 d et des coulisseaux 2a, 2b, 2c et 2d. Seul le module c a son coulisseau en position odorante, c'est-à-dire en position telle que sa cartouche d'odeur se trouve en regard de la fenêtre 8c du carter correspondant 1 c. Les trois autres coulisseaux sont tous en configuration de non diffusion d'odeur car ce sont leurs fenêtres 4a, 4b et 4d qui se trouvent en regard des fenêtres 8a, 8b, 8d des carters 1 a, 1b et 1 d correspondant. C'est donc l'odeur de la cartouche 10c et uniquement celle-ci qui est diffusée dès lors que circule un flux d'air tel que représenté par les flèches sur la figure 4. Ce flux d'air est créé par la turbine 11 placée à l'intérieur d'un tube 12.

En actionnant les moteurs 5a, 5b, 5c, et 5d l'utilisateur choisit l'odeur à diffuser parmi les quatre possibles. Il peut également choisir de ne diffuser aucune odeur en plaçant l'ensemble des coulisseaux 2a, 2b, 2c, et 2d en position telle qu'aucune des cartouches parmi 10a, 10b, 10c, 10d ne se trouve en regard de la fenêtre 8 du carter correspondant. L'activation des moteurs peut être commandée par un automate électronique ou un port électronique de dispositif informatique classique non représenté sur les figures. Ainsi les différentes odeurs présentes dans les cartouches sont diffusées via le même couloir d'air et sortent par la même extrémité.

Lorsqu'un coulisseau 2 se trouve en position ne diffusant pas d'odeur, la cartouche 10 se trouve enfermée de manière inaccessible à l'air, ce qui évite son dessèchement intempestif. Cet enfermement des cartouches non utilisées s'obtient aisément en juxtaposant les modules au contact les uns des autres, le carter de chaque module se plaçant au contact du carter du module voisin. Une étanchéité suffisante sera obtenue, tout en laissant les coulisseaux libres de changer de position, si l'on prévoit par conception un jeu mécanique de faible dimension, par exemple de l'ordre du dixième de millimètre, entre un coulisseau et le carter du module voisin. Le module a n'ayant pas de voisin sur la face où se trouve visible la cartouche 10a, une plaque 13 est prévue pour éviter le dessèchement intempestif de la cartouche 10a lorsque le coulisseau 2a est en position non odorante.

Cette configuration en quatre modules n'est qu'un exemple de conception. L'invention permet de concevoir des dispositifs dont le nombre de modules peut être choisi en fonction du nombre d'odeurs différentes que l'on souhaite pouvoir diffuser.

Ce nombre de modules peut également être choisi en fonction de la durée nécessaire de fonctionnement du dispositif avant de devoir ré-imbiber ou remplacer les cartouches d'odeurs. Ainsi, s'il est prévu de diffuser l'une des odeurs plus longuement que les autres, par exemple deux fois plus longuement, le nombre de modules sera avantageusement choisi de manière à pouvoir y placer deux fois plus de cartouches de l'odeur correspondante.

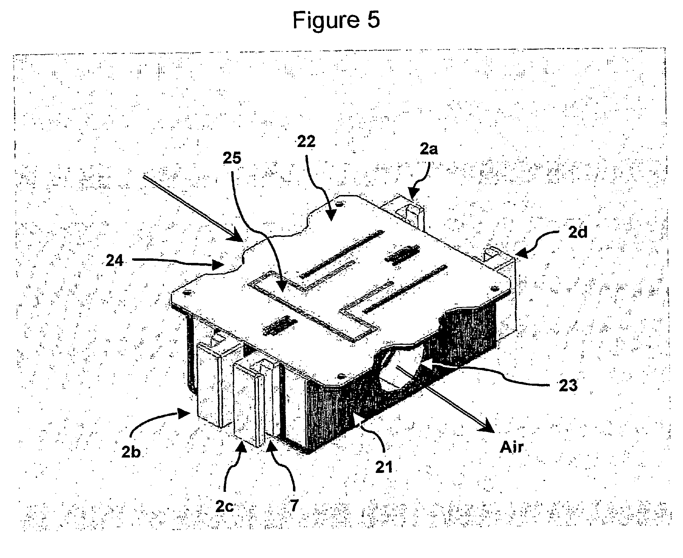

La figure 5 représente une conception d'un ensemble modulaire amovible composé de quatre modules a, b, c et d hébergés dans un boîtier en métal. Le boîtier se compose d'une partie inférieure 21 transversale de forme sensiblement en " U " et d'une partie supérieure 22 de dimensions semblables à celle de la partie inférieure 21 de sorte à former ensemble sensiblement un parallélépipède pour la réception des modules. Les parois latérales de la partie inférieure 21 comprennent chacune une ouverture 23 correspondant à l'ouverture 8 des modules afin de permettre le passage du flux d'air. Éventuellement la partie inférieure 21 comprend des rebords sur les parties hautes du " U " pour permettre la fixation de la partie supérieure (2 ainsi que des encoches 24 sur ces rebords (et de façon symétrique sur la partie supérieure 22) pour permettre la manipulation du dispositif.

Les quatre modules sont alignés de telle sorte que leurs ouvertures 8 correspondent avec les ouvertures 23 pratiquées dans le boîtier du dispositif. En position fermée, seules les rainures 7 des coulisseaux dépassent du boîtier. Afin d'optimiser la place occupée par les servomoteurs 5, deux modules possèdent leur rainure 7 d'un côté du dispositif et les deux autres modules de l'autre côté. En référence à la figure 5, les deux coulisseaux extrêmes (a et 2d ont leur rainure 7 du même côté et se faisant face de telle sorte que les servomoteurs 5a et 5d associés puissent être placés dans l'espace laissé libre à l'arrière (côté où ne se trouvent pas les rainures) des deux autres modules b et c : le servomoteur 5a se trouvant sensiblement à l'arrière du module b.

De même, les rainures des modules coulisseaux 2b et 2c dépassent du même côté du boîtier et sont dos-à-dos de telle sorte que le servomoteur 5b du coulisseau 2b soit sensiblement disposé à l'arrière du module a.

La partie supérieure 22 du boîtier comprend une languette 25 usinée dans la masse, de forme en " T " placée au-dessus des quatre modules et dont la largeur est sensiblement égale à la largeur des quatre modules mis côte à côte dans le boîtier. La base du " T " n'est pas usinée reliant de la sorte la languette 25 à la masse de la partie supérieure 22. La partie haute de la languette 25 est libre d'un mouvement léger vers le haut ou le bas.

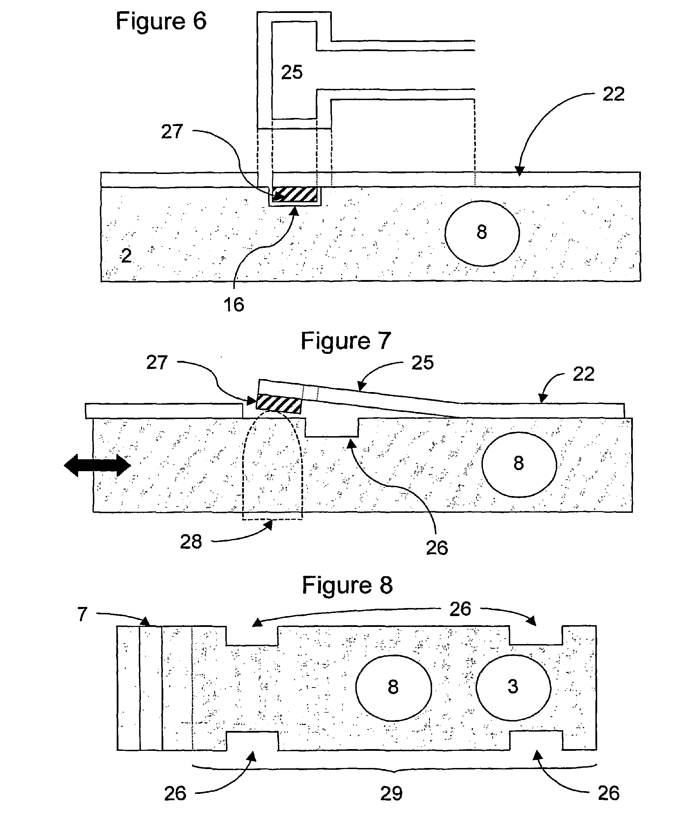

En référence aux figures 6 et 7, les coulisseaux 2 sont également munis d'une encoche 26 sur la tranche faisant face à la languette 25 dans leur position non odorante et la languette 25 est munie sur sa largeur (barre du " T ") d'un ergot 27 à l'intérieur du boîtier. En position fermée illustrée par la figure 6, l'ergot 27 de la languette 25 prend place dans l'encoche 26 du coulisseau 2 le bloquant ainsi de toute ouverture. En position ouverte illustrée par la figure 7, la languette 25 est relevée, l'ergot 27 n'est plus dans l'encoche 26 du coulisseau. Ce dernier est alors libre de coulisser dans le module.

Ces moyens de blocage 25, 26, 27 sont utilisés pour maintenir fermés les coulisseaux 2 en position non odorante lorsque l'ensemble modulaire est extrait de son emplacement d'utilisation. La position ouverte des moyens de blocage est obtenue par la présence d'un " aileron " métallique 28 sur l'emplacement du dispositif de diffusion d'odeurs destiné à recevoir cet ensemble modulaire. L'aileron 28, de faible épaisseur et d'une hauteur supérieure à la hauteur de la partie basse 22 du boîtier, se glisse entre les coulisseaux 2b et 2c pour venir pousser la languette 25.

Dans un mode de réalisation amélioré, il est envisagé que cet aileron 28 soit mobile et commandé par des moyens de pilotage qui bloquent les coulisseaux lorsque l'ensemble modulaire n'est pas utilisé (pas inséré dans le dispositif de diffusion) et les rend libres lors de la mise sous tension, par exemple, du dispositif.

Au vu de la dissymétrie (encoche de blocage, distance moyens de blocage-rainure,..) de l'ensemble modulaire proposé sur la figure 5, il est intéressant de fournir un unique coulisseau qui puisse être utilisé pour l'ensemble des modules. La figure 8 propose une solution de coulisseau présentant la fenêtre 8 au milieu de la partie 29 accueillie par le boîtier de l'ensemble modulaire, une fenêtre 3 d'accueil de la cartouche située du côté opposé à la rainure 7 et quatre encoches 26 dans les tranches supérieures et inférieures du coulisseau, ces encoches étant symétriques les unes des autres dans la partie 29 pour faire face à la languette 25 quel que soit le sens d'introduction du coulisseau dans le module.

Dans un mode de réalisation plus sophistiquée de l'invention, le coulisseau comprend plusieurs fenêtres 3 ce qui permet d'y placer plusieurs cartouches d'odeurs. Dans ce cas les éléments des modules, et notamment le moyen 5, sont adaptés afin de pouvoir positionner l'une ou l'autre des fenêtres 3 ainsi que la fenêtre 4 en regard de la fenêtre 8 du carter 1.

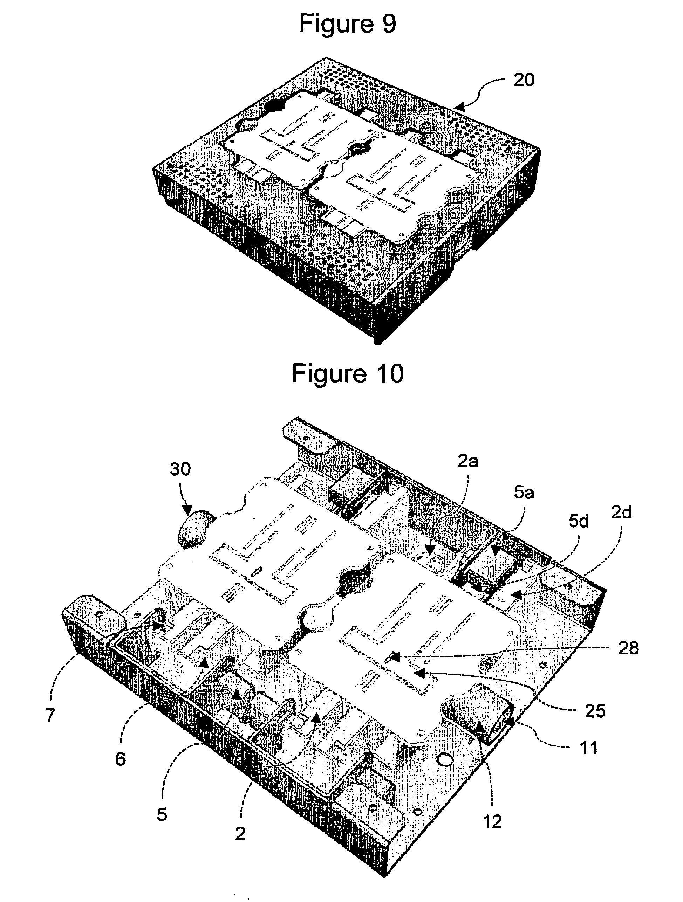

Les figures 9 et 10 représentent une conception de dispositif de diffusion d'odeurs destiné à recevoir deux ensembles modulaires de quatre modules précédemment décrits en rapport avec la figure 5. L'appareil comprend un capot 20 muni d'une ouverture et d'encoches pour permettre la mise en place et le retrait des deux blocs de modules. Il est à noter que la forme de l'ouverture ne permet le retrait de ces blocs que si les coulisseaux 2 sont tous en position non odorante, c'est-à-dire repliés au maximum à l'intérieur des carters 1.

Les deux ensembles (blocs) modulaires et leurs modules intérieurs sont alignés afin que leurs ouvertures 8 correspondent avec la turbine 11, le tube/chemin d'air 12 et la sortie 30 du dispositif. Lors de l'insertion des ensembles modulaires, les ailerons 28 soulèvent la languette 25 laissant libres de coulisser les coulisseaux 2.

Les servomoteurs 5a et 5d situés à l'arrière des modules b et c pour actionner les coulisseaux 2a et 2d sont disposés l'un sur l'autre.

En juxtaposant plusieurs ensembles modulaires, ce dispositif de diffusion d'odeurs permet d'accroître très nettement le nombre d'odeurs pouvant être pilotées.

L'invention porte également sur le procédé de diffusion d'odeurs mettant en oeuvre l'un des dispositifs décrits précédemment. Ce procédé consiste une étape d'insertion de cartouches d'odeurs 10 à l'intérieur des fenêtres 3 des modules; une étape de création d'un flux d'air au moyen de la turbine électrique 11, ledit flux d'air traversant l'ensemble des fenêtres 8 des carters 1; une étape de présentation d'au moins une fenêtre 3 en vis-à-vis de la fenêtre 8 de telle sorte que le flux d'air traversant les fenêtres 8 traverse également la cartouche d'odeur 10 contenue dans ladite fenêtre 3 présentée ce qui diffuse l'odeur de celle-ci.

La cartouche 10 amovible contient des matériaux imbibés de molécules odorantes volatiles, destinée à être insérée dans les fenêtres 3 des modules décrits plus haut. Cette cartouche présente notamment :
- deux surfaces en vis-à-vis conçues de manière à laisser le libre passage d'un flux d'air tout en emprisonnant les matériaux,
- une épaisseur sensiblement inférieure à celle du coulisseau 2 afin de permettre le libre coulissement de celui-ci dans le carter 1 y compris lorsque la cartouche 10 est en place dans la fenêtre 3 du coulisseau 2,
- une forme épousant sensiblement celle de la fenêtre 3 de manière à ce que la totalité du flux d'air traversant la fenêtre 3 traverse la cartouche 10,
- une dimension en largeur légèrement supérieure à celle de la fenêtre 3 de manière à ce que la cartouche 10 s'insère et s'enlève avec une légère force en jouant sur l'élasticité de la cartouche 10.

La cartouche est également aimantée au moyen d'une structure rigide entourant le matériau imbibé et venant épouser la forme de la fenêtre 3. La structure rigide aimantée permet une installation facile de la cartouche 10 et un maintien de celle-ci dans la fenêtre 3.

Typiquement, des matériaux utilisés pour de telles cartouches sont de nature polymère, par exemple le PEBAX (Polyether Block Amides de la société ATOFINA - noms commerciaux).

Dans un mode de réalisation particulier, des puces électroniques à lecture par contact sont intégrées aux modules ou aux ensembles modulaires. Le dispositif accueillant les modules présente des lecteurs à contact de puces électroniques, ces lecteurs faisant face aux puces des modules lorsque ceux-ci sont introduits dans le dispositif de diffusion d'odeurs.

Les puces permettent de renseigner un grand nombre d'informations utilisées dans la gestion des modules :
- date de chargement du module sur le dispositif ;
- odeur dans le module/les modules ;
- l'emplacement des odeurs dans un ensemble de modules ;
- la durée de vie, la durée d'utilisation.

Selon le mode de réalisation choisi, une puce peut être placée sur la face inférieure du module. À chaque chargement d'un nouveau module dans le dispositif, le dispositif de diffusion lit la puce (les informations du nouveau module inséré) et se reparamètre avec les informations du nouveau module chargé (mise à jour de paramètres, par exemple).

Selon un autre mode de réalisation, une puce est présente sur la tranche inférieure du carter 1 de chacun des modules a, b, c et d, la face inférieure du boîtier présentant alors quatre ouvertures au niveau des puces des modules afin d'en permettre la lecture par les lecteurs de l'appareil. La puce peut être préparamétrée lors sa conception, le module correspondant étant alors uniquement chargé dans le dispositif. La puce peut également être paramétrée, par l'installateur et/ou automatiquement par les lecteurs de l'appareil réceptionnant le dispositif, lors du chargement du module en renseignant, par exemple, la date de chargement et l'emplacement du module concerné.

Les données présentes dans la puce peuvent ainsi être utilisées par le logiciel de pilotage des dispositifs pour, selon l'encodage dans le flux multimédia correspondant auquel on souhaite associer une odeur, activer le bon coulisseau. En présence des puces, la gestion des modules est améliorée :
- la connaissance de la date de chargement permet d'avertir l'utilisateur du vieillissement de sa cartouche ;
- des informations sur le temps d'utilisation d'une cartouche permettent à la fois d'adapter le flux d'air (pour tenir compte de l'appauvrissement en molécules dans le temps de la cartouche) et d'indiquer que la cartouche n'est plus efficace (temps total d'utilisation dépassé) ;
- le logiciel de pilotage utilise la nature des odeurs des modules et le temps d'utilisation de leur cartouche avec des abaques contenus dans une base de données afin d'évaluer la consommation des molécules d'odeurs lors de l'utilisation et de la non-utilisation des cartouches ;
- lorsqu'un dispositif est introduit dans un appareil de pilotage, typiquement un tableau animé multisensoriel, la détection des odeurs présentes et de leur emplacement est réalisée automatiquement ; les informations sont remontées au logiciel de pilotage qui peut activer de façon sûre les bons servomoteurs.

## Revendications

1. Dispositif de diffusion d'odeurs comprenant des moyens de génération d'un flux d'air (11) et une sortie (30), lesdits moyens de génération et la sortie définissant entre eux un chemin pour le flux d'air, **caractérisé en ce qu'**il comprend :
- une pluralité de modules de diffusion d'odeur, chaque module comprenant :
- un carter (1) dans lequel est pratiquée une fenêtre (8),
- un coulisseau (2), dans lequel sont pratiquées :
- au moins une première fenêtre (3) destinée à accueillir une source de molécules odorantes (10), et
- une deuxième fenêtre (4) ;
le coulisseau coulissant à l'intérieur du carter (1) entre :
- une position de diffusion dans laquelle ladite première fenêtre (3) du coulisseau est en regard de la fenêtre (8) du carter (1), et
- une position non odorante dans laquelle ladite deuxième fenêtre (4) du coulisseau est en regard de la fenêtre (8) du carter (1) ;
les modules étant positionnés de manière à ce que les axes des fenêtres (8) des carters (1) des différents modules soient alignés sur ledit chemin pour le flux d'air ; et
- des moyens (5) permettant d'amener individuellement les coulisseaux (2) sur l'une desdites positions ;
de sorte que le flux d'air traverse successivement chaque module par la fenêtre (8) du carter (1) ainsi que par l'une des première et deuxième fenêtres du coulisseau (2) selon la position de celui-ci.

2. Dispositif de diffusion d'odeurs selon la revendication 1, **caractérisé en ce que** ledit chemin présente des parois formant un
couloir d'air (12) et lesdites fenêtres (8) desdits carters étant alignées dans le couloir d'air.

3. Dispositif de diffusion d'odeurs selon la revendication précédente, **caractérisé en ce que** lesdites fenêtres (3, 4, 8) sont sensiblement de la dimension de la section dudit couloir d'air et sont orientées sensiblement perpendiculairement à l'axe de ce couloir.

4. Dispositif de diffusion d'odeurs selon l'une quelconque des revendications précédentes, **caractérisé en ce que** lesdits modules sont juxtaposés carter contre carter.

5. Dispositif de diffusion d'odeurs selon l'une quelconque des revendications précédentes, **caractérisé en ce que** les moyens (5) permettant de déplacer les coulisseaux (2) sont actionnés par un organe de commande électrique.

6. Dispositif de diffusion d'odeurs selon l'une quelconque des revendications précédentes, **caractérisé en ce qu'**il comprend une pluralité de moyens (5) permettant de déplacer les coulisseaux (2), chacun de ladite pluralité de moyens (5) étant associé individuellement à l'un de ladite pluralité de modules de diffusion d'odeurs.

7. Dispositif de diffusion d'odeurs selon la revendication précédente, **caractérisé en ce que** les moyens (5) permettant de déplacer le coulisseau (2) sont un servomoteur dont le stator est solidaire du carter (1) et dont le rotor est solidaire d'un doigt excentré (6) qui coopère avec une rainure (7) pratiquée dans le coulisseau (2).

8. Dispositif de diffusion d'odeurs selon l'une quelconque des revendications précédentes, **caractérisé en ce que** chacun des carters (1) présente globalement une forme en U à l'intérieur de laquelle s'insère et coulisse le coulisseau (2) associé au carter.

9. Dispositif de diffusion d'odeurs selon l'une quelconque des revendications précédentes, **caractérisé en ce que** chaque module n'ayant pas de voisin sur la face où se trouve visible la source de molécules odorantes comprend une plaque (13) apte à enfermer de manière inaccessible à l'air la source (10) lorsque le coulisseau (2) se trouve en position telle que la deuxième fenêtre (4) se trouve en regard de la fenêtre (8) du carter (1).

10. Dispositif de diffusion d'odeurs selon l'une quelconque des revendications précédentes, **caractérisé en ce qu'**au moins un module est amovible et comprend des moyens de blocage (25, 26, 27) des coulisseaux (2) dans la position non odorante lorsqu'il n'est pas inséré dans le dispositif et **en ce que** ledit dispositif comprend des moyens de déblocage (28) desdits moyens de blocage lorsque le module amovible est inséré dans le dispositif.

11. Dispositif de diffusion d'odeurs selon l'une quelconque des revendications précédentes, **caractérisé en ce que** lesdits modules comportent des moyens de stockage de données comprenant au moins des données numériques représentatives d'informations concernant les sources (10) de molécules odorantes qu'ils renferment, et le dispositif comprend, en outre, des moyens de lecture desdits moyens de stockage de données, ces moyens de lecture étant reliés à une carte électronique de pilotage du dispositif.

12. Dispositif de diffusion d'odeurs selon l'une quelconque des revendications précédentes, **caractérisé en ce que** ladite source (10) de molécules odorantes est une cartouche amovible imbibée de molécules odorantes.

13. Dispositif de diffusion d'odeurs selon la revendication précédente, **caractérisé en ce que** ladite cartouche (10) comprend des moyens de stockage de données comprenant au moins des données numériques représentatives de l'odeur contenue dans la cartouche et le dispositif comprend, en outre, des moyens de lecture desdits moyens de stockage de données, ces moyens de lecture étant reliés à une carte électronique de pilotage du dispositif.

14. Dispositif de diffusion d'odeurs selon l'une quelconque des revendications précédentes, **caractérisé en ce que** lesdits moyens de génération d'un flux d'air (11) sont choisis parmi un ventilateur et une turbine à air.

15. Dispositif de diffusion d'odeurs selon l'une quelconque des revendications précédentes, **caractérisé en ce qu'**il comprend, en outre, des moyens d'accélération du flux d'air disposés dans le couloir d'air et pilotés par une carte électronique de pilotage du dispositif.

16. Dispositif de diffusion d'odeurs selon l'une quelconque des revendications précédentes, **caractérisé en ce qu'**il comprend, en outre, des moyens d'adaptation du débit du flux d'air en sortie dudit couloir d'air.

17. Dispositif de diffusion d'odeurs selon l'une quelconque des revendications précédentes, **caractérisé en ce que** le coulisseau (2) d'au moins un module comprend, en outre, au moins une troisième fenêtre agencée pour accueillir une deuxième source de molécules odorantes (IO), lesdits moyens (5) permettant de déplacer le coulisseau étant alors aptes à amener le coulisseau sur l'une des au moins trois positions correspondant à l'alignement d'une fenêtre du coulisseau avec la fenêtre (8) du carter (1).

18. Dispositif de diffusion d'odeurs selon l'une quelconques des revendications précédentes, **caractérisé en ce qu'**il comprend en outre une structure rigide (21, 22) comprenant des moyens de réception agencés pour disposer 2, 4 ou 8 modules de diffusion d'odeurs dans la structure rigide, la structure rigide étant agencée pour coopérer avec les moyens de réception de telle sorte que les fenêtres (8) des modules de diffusion d'odeurs soient alignées avec un conduit d'air pourvu dans le dispositif, pour former un ensemble modulaire.

19. Dispositif de diffusion d'odeurs selon la revendication précédente, **caractérisé en ce que** l'ensemble modulaire comprend des moyens de blocage (25, 26, 27) agencés pour bloquer les coulisseaux dans la position non odorante lorsque ledit ensemble n'est pas inséré dans le dispositif et pour débloquer lesdits coulisseaux lorsqu'ils coopèrent avec des moyens de déblocage (28) pourvus sur ledit dispositif.

20. Procédé de diffusion d'odeurs mettant en oeuvre un dispositif selon l'une quelconque des revendications 1 à 19, **caractérisé en ce qu'**il comprend :
- une étape d'insertion de cartouches d'odeur (10) à l'intérieur des premières fenêtres (3) des modules,
- une étape de création d'un flux d'air au moyen desdits moyens de production d'un flux d'air (11), ledit flux d'air traversant l'ensemble des fenêtres (8) des carters (1),
- une étape de présentation d'au moins une première fenêtre (3)
en vis-à-vis de la fenêtre (8) d'au moins un carter de telle sorte que le flux d'air traversant les fenêtres (8) des carters traverse également la cartouche d'odeur (10) contenue dans ladite première fenêtre (3) présentée ce qui diffuse l'odeur de celle-ci.

21. Procédé de diffusion d'odeurs selon la revendication précédente, **caractérisé en ce qu'**il comprend, en outre et lors de l'utilisation d'une cartouche (10), une étape de mise à jour d'une puce électronique associée à ladite cartouche et comprenant au moins des données numériques représentatives de l'utilisation de la cartouche (10).

## Claims

1. Device for diffusion of odours, comprising means (11) for generating a flow of air and an outlet (30), said generating means and the outlet defining between them a path for the flow of air, **characterized in that** it comprises:
- a plurality of modules for diffusion of odours, each module comprising:
- a casing (1) in which a window (8) is formed,
- a slide (2), in which are formed:
- at least one first window (3) intended to receive a source (10) of odourizing molecules, and
- a second window (4);
the slide sliding inside the casing (1) between:
- a diffusing position in which said first window (3) of the slide is opposite the window (8) of the casing (1); and
- a non-odourizing position in which said second window (4) of the slide is opposite the window (8) of the casing (1);
the modules being positioned so that the axes of the windows (8) of the casings (1) of the various modules are aligned on said path for the flow of air; and
- means (5) for moving the slides (2) individually to one of said positions;
so that the flow of air passes successively through each module via the window (8) of the casing (1) and via one of the first and second windows of the slide (2) according to the position of the latter.

2. Device for diffusion of odours according to Claim 1, **characterized in that** said path has walls forming an air corridor (12) and said windows (8) of said casings being aligned in the air corridor.

3. Device for diffusion of odours according to the preceding claim, **characterized in that** said windows (3, 4, 8) are substantially the same size as the section of said air corridor and are oriented substantially perpendicularly to the axis of that corridor.

4. Device for diffusion of odours according to any of the preceding claims, **characterized in that** said modules are juxtaposed casing against casing.

5. Device for diffusion of odours according to any one of the preceding claims, **characterized in that** the means (5) for moving the slides (2) are actuated by an electrical control unit.

6. Device for diffusion of odours according to any one of the preceding claims, **characterized in that** it comprises a plurality of means (5) for moving the slides (2), each of said plurality of means (5) being individually associated with one of said plurality of odour diffusing modules.

7. Device for diffusion of odours according to the preceding claim, **characterized in that** the means (5) for moving the slide (2) consist of a servomotor the stator of which is fastened to the casing (1) and the rotor of which is fastened to an eccentric finger (6) that cooperates with a groove (7) formed in the slide (2) .

8. Device for diffusion of odours according to any one of the preceding claims, **characterized in that** each of the casings (1) is generally U-shaped inside which shape the slide (2) associated with the casing is inserted and slides.

9. Device for diffusion of odours according to any one of the preceding claims, **characterized in that** each module having no neighbour on the side on which the source of odourizing molecules is visible comprises a plate (13) adapted to close the source (10) in a manner inaccessible to air when the slide (2) is in a position such that the second window (4) is opposite the window (8) of the casing (1).

10. Device for diffusion of odours according to any one of the preceding claims, **characterized in that** at least one module is removable and comprises means (25, 26, 27) for locking the slides (2) in the non-odourizing position when it is not inserted in the device and **in that** said device comprises means (28) for unlocking said locking means when the removable module is inserted in the device.

11. Device for diffusion of odours according to any one of the preceding claims, **characterized in that** said modules include data storage means containing at least digital data representative of information concerning the sources (10) of odourizing modules that they contain, and the device further comprises means for reading said data storage means, these reading means being connected to an electronic control card of the device.

12. Device for diffusion of odours according to any one of the preceding claims, **characterized in that** said source (10) of odourizing molecules is a removable cartridge soaked with odorizing molecules.

13. Device for diffusion of odours according to the preceding claim, **characterized in that** said cartridge (10) comprises data storage means containing at least digital data representative of the odour contained in the cartridge and the device further comprises means for reading said data storage means, these reading means being connected to an electronic control card of the device.

14. Device for diffusion of odours according to any one of the preceding claims, **characterized in that** said means (11) for generating a flow of air are chosen from a fan and an air turbine.

15. Device for diffusion of odours according to any one of the preceding claims, **characterized in that** it further comprises means for accelerating the flow of air disposed in the air corridor and controlled by an electronic control card of the device.

16. Device for diffusion of odours according to any one of the preceding claims, **characterized in that** it further comprises means for adaptation of the flow rate of the flow of air at the outlet of said air corridor.

17. Device for diffusion of odours according to any one of the preceding claims, **characterized in that** the slide (2) of at least one module further comprises at least a third window adapted to receive a second source (10) of odourizing molecules, said means (5) for moving movement of the slide then being adapted to move the slide to one of the at least three positions corresponding to the alignment of a window of the slide with the window (8) of the casing (1).

18. Device for diffusion of odours according to any one of the preceding claims, **characterized in that** it further comprises a rigid structure (21, 22) comprising receiving means adapted to dispose two, four or eight modules for diffusion of odours in the rigid structure, the rigid structure being adapted to cooperate with the receiving means so that the windows (8) of the modules for diffusion of odours are aligned with an air duct provided in the device to form a modular assembly.

19. Device for diffusion of odours according to the preceding claim, **characterized in that** the modular assembly comprises locking means (25, 26, 27) adapted to lock the slides in the non-odourizing position when said assembly is not inserted in the device and to unlock said slides when they cooperate with unlocking means (28) provided on said device.

20. Method of diffusion of odours using a device according to any one of Claims 1 to 19, **characterized in that** it comprises:
- a step of inserting odour cartridges (10) inside the first windows (3) of the modules,
- a step of creating a flow of air by means of said means (11) for producing a flow of air, said flow of air passing through all of the windows (8) of the casings (1),
- a step of presenting at least one first window (3) facing the window (8) of at least one casing so that the flow of air passing through the window (8) of the casings also passes through the odour cartridge (10) contained in said first window (3) which diffuses the odour thereof.

21. Method of diffusion of odours according to the preceding claim, **characterized in that** it further comprises, during the use of a cartridge (10), a step of updating an electronic chip associated with said cartridge and comprising at least digital data representative of the use of the cartridge (10).

## Patentansprüche

1. Vorrichtung zur Abgabe von Düften, umfassend Mittel zur Erzeugung eines Luftstroms (11) und einen Ausgang (30), wobei die Mittel zur Erzeugung und der Ausgang zwischen sich einen Weg für den Luftstrom definieren, **dadurch gekennzeichnet, dass** sie Folgendes umfasst:
- eine Vielzahl von Duftabgabemodulen, wobei jedes Modul Folgendes umfasst:
- ein Gehäuse (1), in dem ein Fenster (8) vorgesehen ist,
- einen Gleitschieber (2), in dem vorgesehen sind:
- mindestens ein erstes Fenster (3), das dazu bestimmt ist, eine Quelle von duftenden Molekülen (10) aufzunehmen, und
- ein zweites Fenster (4);
wobei der Gleitschieber im Inneren des Gehäuses (1) gleitet zwischen:
- einer Abgabeposition, in der das erste Fenster (3) des Gleitschiebers gegenüber dem Fenster (8) des Gehäuses (1) ist, und
- einer nicht duftenden Position, in der das zweite Fenster (4) des Gleitschiebers gegenüber dem Fenster (8) des Gehäuses (1) ist;
wobei die Module derart positioniert sind, dass die Achsen der Fenster (8) der Gehäuse (1) der verschiedenen Module auf dem Weg für den Luftstrom ausgerichtet sind; und
- Mittel (5), die es ermöglichen, die Gleitschieber (2) individuell in eine der Positionen zu bringen;
so dass der Luftstrom nacheinander durch jedes Modul durch das Fenster (8) des Gehäuses (1) sowie durch eines der ersten und zweiten Fenster des Gleitschiebers (2) je nach Position desselben hindurchströmt.

2. Vorrichtung zur Abgabe von Düften nach Anspruch 1, **dadurch gekennzeichnet, dass** der Weg Wände aufweist, die einen Luftkorridor (12) bilden, wobei die Fenster (8) der Gehäuse in dem Luftkorridor ausgerichtet sind.

3. Vorrichtung zur Abgabe von Düften nach dem vorhergehenden Anspruch, **dadurch gekennzeichnet, dass** die Fenster (3, 4, 8) im Wesentlichen die Abmessung des Querschnitts des Luftkorridors haben und im Wesentlichen senkrecht auf die Achse dieses Korridors ausgerichtet sind.

4. Vorrichtung zur Abgabe von Düften nach einem der vorhergehenden Ansprüche, **dadurch gekennzeichnet, dass** die Module Gehäuse an Gehäuse aneinander angrenzen.

5. Vorrichtung zur Abgabe von Düften nach einem der vorhergehenden Ansprüche, **dadurch gekennzeichnet, dass** die Mittel (5), die es ermöglichen, die Gleitschieber (2) zu verschieben, durch ein elektrisches Steuerelement betätigt werden.

6. Vorrichtung zur Abgabe von Düften nach einem der vorhergehenden Ansprüche, **dadurch gekennzeichnet, dass** sie eine Vielzahl von Mitteln (5) umfasst, die es ermöglichen, die Gleitschieber (2) zu verschieben, wobei die Vielzahl von Mitteln (5) individuell mit einem der Vielzahl von Duftabgabemodulen verbunden ist.

7. Vorrichtung zur Abgabe von Düften nach dem vorhergehenden Anspruch, **dadurch gekennzeichnet, dass** die Mittel (5), die es ermöglichen, den Gleitschieber (2) zu verschieben, ein Servomotor sind, dessen Stator mit dem Gehäuse (1) verbunden ist, und dessen Rotor mit einem exzentrischen Finger (6) verbunden ist, der mit einer Nut (7) zusammenwirkt, die im Gleitschieber (2) vorgesehen ist.

8. Vorrichtung zur Abgabe von Düften nach einem der vorhergehenden Ansprüche, **dadurch gekennzeichnet, dass** jedes der Gehäuse (1) global eine U-Form aufweist, in die der mit dem Gehäuse verbundene Gleitschieber (2) eingesetzt wird und in die er gleitet.

9. Vorrichtung zur Abgabe von Düften nach einem der vorhergehenden Ansprüche, **dadurch gekennzeichnet, dass** jedes Modul, das auf der Seite, auf der sich die Quelle von duftenden Molekülen befindet, keinen Nachbarn hat, eine Platte (13) umfasst, die geeignet ist, auf für die Luft nicht zugängliche Weise die Quelle (10) einzuschließen, wenn sich der Gleitschieber (2) in einer derartigen Position befindet, dass sich das zweite Fenster (4) gegenüber dem Fenster (8) des Gehäuses (1) befindet.

10. Vorrichtung zur Abgabe von Düften nach einem der vorhergehenden Ansprüche, **dadurch gekennzeichnet, dass** mindestens ein Modul abnehmbar ist und Mittel (25, 26, 27) zur Feststellung der Gleitschieber (2) in der nicht duftenden Position umfasst, wenn es nicht in die Vorrichtung eingesetzt ist, und dass die Vorrichtung Mittel zur Freigabe (28) der Mittel zur Festsellung umfasst, wenn das abnehmbare Modul in die Vorrichtung eingesetzt ist.

11. Vorrichtung zur Abgabe von Düften nach einem der vorhergehenden Ansprüche, **dadurch gekennzeichnet, dass** die Module Mittel zur Datenspeicherung umfassen, mindestens umfassend digitale Daten, die für Informationen betreffend die Quellen (10) von duftenden Molekülen, die sie einschließen, repräsentativ sind, und dass die Vorrichtung ferner Mittel zum Lesen der Mittel zur Datenspeicherung umfasst, wobei diese Mittel zum Lesen mit einer elektronischen Steuerkarte der Vorrichtung verbunden sind.

12. Vorrichtung zur Abgabe von Düften nach einem der vorhergehenden Ansprüche, **dadurch gekennzeichnet, dass** die Quelle (10) von duftenden Molekülen eine abnehmbare Kartusche ist, die mit duftenden Molekülen getränkt ist.

13. Vorrichtung zur Abgabe von Düften nach dem vorhergehenden Anspruch, **dadurch gekennzeichnet, dass** die Kartusche (10) Mittel zur Datenspeicherung umfasst, mindestens umfassend digitale Daten, die für den in der Kartusche enthaltenen Duft repräsentativ sind, und dass die Vorrichtung ferner Mittel zum Lesen der Mittel zur Datenspeichenung umfasst, wobei diese Mittel zum Lesen mit einer elektronischen Steuerkarte der Vorrichtung verbunden sind.

14. Vorrichtung zur Abgabe von Düften nach einem der vorhergehenden Ansprüche, **dadurch gekennzeichnet, dass** die Mittel zur Erzeugung eines Luftstroms (11) unter einem Ventilator und einer Luftturbine ausgewählt werden.

15. Vorrichtung zur Abgabe von Düften nach einem der vorhergehenden Ansprüche, **dadurch gekennzeichnet, dass** sie ferner Mittel zur Beschleunigung des Luftstroms umfasst, die in dem Luftkorridor angeordnet sind und von einer elektronischen Steuerkarte der Vorrichtung gesteuert werden.

16. Vorrichtung zur Abgabe von Düften nach einem der vorhergehenden Ansprüche, **dadurch gekennzeichnet, dass** sie ferner Mittel zur Anpassung der Menge des Luftstroms am Ausgang des Luftkorridors umfasst.

17. Vorrichtung zur Abgabe von Düften nach einem der vorhergehenden Ansprüche, **dadurch gekennzeichnet, dass** der Gleitschieber (2) mindestens eines Moduls ferner mindestens ein drittes Fenster umfasst, das dazu vorgesehen ist, eine zweite Duftmolekülquelle (IO) aufzunehmen, wobei die Mittel (5), die es ermöglichen, den Gleitschieber zu verschieben, nun geeignet sind, den Gleitschieber in eine der mindestens drei Positionen, die der Ausrichtung eines Fensters des Gleitschiebers mit dem Fenster (8) des Gehäuses (1) entsprechen, zu bringen.

18. Vorrichtung zur Abgabe von Düften nach einem der vorhergehenden Ansprüche, **dadurch gekennzeichnet, dass** sie ferner eine starre Struktur (21, 22) besitzt, umfassend Aufnahmemittel, die derart angeordnet sind, dass sie 2, 4 oder 8 Duftabgabemodule in der starren Struktur anordnen, wobei die starre Struktur derart vorgesehen ist, dass sie mit den Aufnahmemitteln derart zusammenwirkt, dass die Fenster (8) der Duftabgabemodule mit einer Luftleitung, die in der Vorrichtung vorgesehen ist, ausgerichtet werden,
um eine modulare Einheit zu bilden.

19. Vorrichtung zur Abgabe von Düften nach dem vorhergehenden Anspruch, **dadurch gekennzeichnet, dass** die modulare Einheit Mittel zur Feststellung (25, 26, 27) umfasst, die derart angeordnet sind, dass sie die Gleitschieber in der nicht duftenden Position feststellen, wenn die Einheit nicht in die Vorrichtung eingesetzt ist, und dass sie die Gleitschieber freigeben, wenn sie mit Mitteln zur Freigabe (28), die auf der Vorrichtung vorgesehen sind, zusammenwirken.

20. Verfahren zur Abgabe von Düften, das eine Vorrichtung nach einem der Ansprüche 1 bis 19 einsetzt, **dadurch gekennzeichnet, dass** es Folgendes umfasst:
- einen Schritt des Einsetzens von Duftkartuschen (10) in das Innere der ersten Fenster (3) der Module,
- einen Schritt der Erzeugung eines Luftstroms mit Hilfe der Mittel zur Erzeugung eines Luftstroms (11), wobei der Luftstrom durch die Gesamtheit der Fenster (8) der Gehäuse (1) strömt,
- einen Schritt der Präsentation mindestens eines ersten Fensters (3) gegenüber dem Fenster (8) mindestens eines Gehäuses, so dass der durch die Fenster (8) der Gehäuse strömende Luftstrom auch durch die Duftkartusche (10) strömt, die in dem ersten präsentierten Fenster (3) enthalten ist, wodurch der Duft derselben verbreitet wird.

21. Verfahren zur Abgabe von Düften nach dem vorhergehenden Anspruch, **dadurch gekennzeichnet, dass** es ferner und bei Verwendung einer Kartusche (10) einen Schritt der Aktualisierung eines elektronischen Chips umfasst, der mit der Kartusche verbunden ist und mindestens für die Verwendung der Kartusche (10) repräsentative digitale Daten umfasst.
